# FASCICULE DE BREVET EUROPEEN

(11) **EP 4 347 545 B1**
(45) Date de publication et mention de la délivrance du brevet: **04.06.2025**
(21) Numéro de dépôt: 22732300.3
(22) Date de dépôt: 27.05.2022
(51) Int. Cl.: C07C 45/38, C07C 47/21

(54) **NOUVEAU PROCÉDÉ DE FABRICATION DE PHÉROMONE EN CONTINU**
NEUES VERFAHREN ZUR KONTINUIERLICHEN PHEROMONPRODUKTION
NOVEL PROCESS FOR CONTINUOUS PHEROMONE PRODUCTION

(30) Priorité: 28.05.2021 FR 2105616
(43) Date de publication de la demande: 10.04.2024
(73) Titulaire: Melchior Material And Life Science France, 64170 LACQ (FR)
(72) Inventeur: GUERRET, Olivier, 46170 PERN (FR); LAUREANO, Hugo, 13200 ARLES (FR)
(74) Mandataire: Regimbeau
(86) Numéro de dépôt international: PCT/FR2022/051001
(87) Numéro de publication internationale: WO 2022/248809

(56) Documents cités:
- EP-A2- 0 318 990
- WO-A2-2018/150379
- CN-A- 110 143 856
- CN-A- 111 253 228
- US-A- 4 228 093
- US-A- 5 118 866
- FUJIWARA KATSUAKI ET AL: "Continuous-Flow Synthesis of Cationic Lipid SST-01 via Safe and Scalable Aerobic Oxidation and Reductive Amination", vol. 24, no. 10, 16 October 2020 (2020-10-16), US, pages 1988 - 1995, XP055874592, ISSN: 1083-6160, Retrieved from the Internet <URL:https://pubs.acs.org/doi/pdf/10.1021/acs.oprd.0c00084> DOI: 10.1021/acs.oprd.0c00084
- VANOYE LAURENT ET AL: "Gas-Liquid Segmented Flow Microfluidics for Screening Copper/TEMPO-Catalyzed Aerobic Oxidation of Primary Alcohols", ADVANCED SYNTHESIS AND CATALYSIS, vol. 357, no. 4, 4 March 2015 (2015-03-04), pages 739 - 746, XP055874588, ISSN: 1615-4150, DOI: 10.1002/adsc.201400925

## Description

Nouveau procédé de synthèse de phéromones à terminaison aldéhyde selon la réaction : 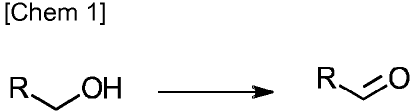 où R est une chaine aliphatique linéaire de formule CₙH₂ₙ₋₂ₚ₊₁ où n désigne un entier naturel supérieur ou égal à 9 et où p est un nombre enter compris entre 1 et 4. Ce procédé est caractérisé par le fait qu'il est réalisé en continu dans un solvant polaire en présence d'un système catalytique à base de cuivre sous une pression d'air supérieure à 1 bar et à une température comprise entre 30 et 200°C. Ce procédé offre l'avantage d'une grande productivité et une grande sélectivité de la réaction.

Les phéromones d'insectes sont les outils de communication spécifiques de chaque espèce. Pour que cette communication soit efficace, il convient que le mélange des composés chimiques, appelé bouquet phéromonal soit très précis. Ainsi, la phéromone sexuelle de la cydalima perspectalis, communément connue sous le nom de pyrale du buis, est un mélange de Z et de E-11 hexadécenal dans le ratio précis de 80/20. En dehors de ce ratio le mâle n'est pas correctement attiré par un attractant. Par ailleurs, des traces de Z /E-11-hexadécenol neutralisent complètement l'effet de la phéromone.

De plus, la synthèse de phéromone pour des applications industrielles s'inscrit dans un cadre réglementaire qui limite drastiquement la possibilité de produire des phéromones avec des impuretés en nombre et en quantité non contrôlés.

Ces deux contraintes induisent pour l'homme du métier des défis technologiques importants, d'autant plus que ces molécules sont utilisées dans des communications ponctuelles entre individus d'une même espèce ce qui induit une dégradation rapide des molécules une fois émises dans l'atmosphère. Cette nature fragile augmente nécessairement l'apparition d'impuretés dans un procédé de synthèse. C'est particulièrement le cas pour les phéromones sexuelles de lépidoptères dont les composants principaux ont des composés de formules générales : 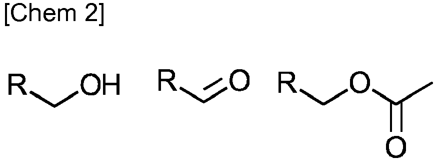 où R est un radical aliphatique linéaire comportant entre 1 et 4 insaturations sous forme de double ou triple liaison conjuguée ou non. La formule générale de ces groupements linéaires R peut donc s'écrire : CₙH₂ₙ₋₂ₚ₊₁ où n est un entier supérieur à 9 et p, qui représente le nombre d'insaturations, est un entier compris entre 1 et 4 (une triple liaison comptant pour 2 insaturations).

Comme l'homme de l'art l'anticipe facilement, les alcool terminaux R-CH₂OH sont les précurseurs de aldéhydes ou des acétates. Si l'acétylation des alcools peut se faire facilement en faisant réagir l'alcool avec un agent d'acétylation tel que l'anhydride acétique ou le chlorure d'acétyle en présence d'une base telle qu'une amine tertiaire, la fabrication des aldéhydes est quant à elle plus compliquée.

En effet les méthodes connues pour obtenir les aldéhydes de cette famille sont tout d'abord des oxydations en présence de peracides organiques (acide chloro-perbenzoique, peracétiques, perpropioniques). Ces oxydants sont agressifs et attaquent aussi les insaturations présentes sur la chaine grasse générant de nombreuses impuretés tel que des époxydes ou des oligomères. Par ailleurs, la suroxydation en acide gras est difficile à éviter. Une autre méthode consiste à utiliser un hypochlorite de sodium ou un hypobromite de sodium en présence d'un nitroxyde ou un sel de nitroxonium. Cette méthode permet d'éviter la suroxydation mais on constate des chlorations ou des bromations des insaturations très gênantes d'un point de vue règlementaire. Pour limiter cet effet, l'eau de javel peut être remplacée par du iodobenzène peracétate mais en sacrifiant l'efficacité économique de la synthèse.

Par ailleurs, tous ces procédés sont des procédés réalisés en batch ce qui nécessite des volumes réactionnels très importants.

Il est donc important pour réussir à industrialiser des phéromones de la forme RCH2O de trouver une technologie d'oxydation productive et sélective.

Seuls deux brevets rapportent des essais de coupler la synthèse de phéromones et les concepts de chimie continue. Le premier travail est rapporté dans le brevet US9,789,455B2. Dans ce brevet, les inventeurs ont décrit un équipement de synthèse continue pour produire entre autres des parfums ou des phéromones. Cet équipement très particulier permet de créer un vortex entre 2 solutions de réactifs injectées en continu.

Le second travail est rapporté dans le brevet US10,071,944B2. Ce brevet rapporte une façon de faire des aldéhydes ou des acides en continu au moyen d'un réacteur continu tubulaire par une réaction d'ozonolyse d'insaturations. Un tel procédé ne saurait s'appliquer à des phéromones porteuses de liaisons multiples sensibles ne conditions oxydantes.

Le monde académique rapporte quelques travaux permettant d'envisager l'utilisation de chimie oxydative en continue pour fabriquer des phéromones. Ainsi, dans V. Liautard & al. Catalysts 2018, 8, 529, les auteurs rapportent le passage du ferrugineol, un alcool secondaire à une cétone en présence de magnésien et d'un aldéhyde donneur selon la réaction d'oxydation d'Oppenauer. Cependant, outre le fait qu'obtenir une cétone est plus facile qu'obtenir un aldéhyde, le rendement dans ce travail est très faible puisque seuls 10% d'alcool sont effectivement convertis en cétone. Par ailleurs la structure de la phéromone ne comporte pas de fonctions chimiques sensibles à des réactions secondaires.

Un autre exemples est aussi trouvé dans K. Fujiwara et al. Org. Process Res. Dev. 2020, 24, 1988-1995.

Une solution envisageable par l'homme du métier aurait pu consister en la transposition des travaux publiés dans L. Vannoye & al. Volume 357, Issue 4, March 9, 2015, pages 739-746. En effet ces derniers ont montré la possibilité de transformer des alcools primaires vinyliques ou aromatiques en utilisant une oxydation aérobie continue en présence d'un catalyseur formé à partir de CuOTf ou Cu(OTf)₂ en présence de bipyridine et de N-méthyl imidazole. Dans le cas des composés aliphatiques linéaires, le système catalytique conduit à de faibles conversions incompatibles avec la pureté nécessaire pour des phéromones. Par ailleurs le catalyseur au cuivre envisagé dans cette publication nécessite un taux de 5% molaire alors que la masse moléculaire du catalyseur est très élevée. Sans un recyclage efficace du catalyseur, les procédés ne peuvent être intéressants d'un point de vue économique mais aussi de leur bilan carbone.

Cette dernière approche si elle pouvait être appliquée avec un catalyseur plus économique serait particulièrement intéressante pour la synthèse de phéromone puisqu'il s'agirait de produire ces phéromones par une oxydations aérobie imitant la respiration des êtres vivants.

La demanderesse a trouvé un procédé nouveau qui est caractérisé en ce que la réaction d'oxydation est réalisée en continu dans un réacteur sous pression d'oxygène, avantageusement dans un réacteur H.E.R (Heat Echange Reactor - réacteur à échange de chaleur), tels que ceux commercialisés par la société Khimod, et qu'il permet de réaliser la réaction suivante en recyclant le catalyseur pour limiter son impact :

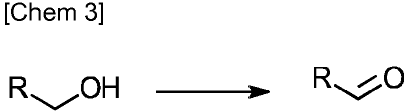

Ainsi, selon un premier mode de réalisation, l'invention concerne un procédé de préparation d'un aldéhyde de formule générale (II) : 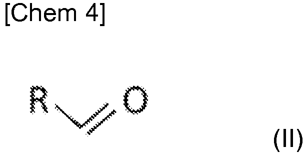
où R est une chaine hydrocarbonée linéaire de formule CₙH₂ₙ₋₂ₚ₊₁ , avec :
   - n qui désigne un entier naturel allant de 9 à 24,
   - p correspondant au nombre d'insaturations de la chaîne hydrocarbonée qui est un nombre entier allant de 1 à 4 ;
ledit procédé est continu et comprend les étapes concomitantes suivantes :
   a. alimenter un réacteur continu sous pression d'oxygène comprise entre 1 et 30 bar avec :
      - un alcool de formule générale (I) : 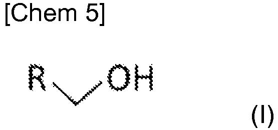 où R, n et p sont tels que définis précédemment pour le composé de formule (II), en solution dans une phase liquide organique apolaire (A) de densité strictement inférieure à 0,7,
      - un catalyseur à base de cuivre en solution dans une phase liquide polaire (B) de densité supérieure ou égale à 0,75,

      les phases (A) et (B) étant non miscibles l'une avec l'autre,
      le ratio molaire alcool/catalyseur à base de cuivre variant de 0,01 à 0,5,
   b. récupérer l'aldéhyde dans la phase (A) par séparation liquide/liquide.

Selon un autre mode de réalisation, le procédé selon l'invention est caractérisé en ce que le catalyseur à base de cuivre comprend en outre au moins un ligand du cuivre de formule générale : 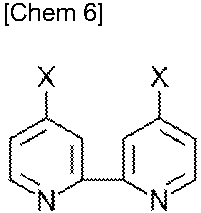 où X est choisi dans le groupe comprenant -C(O)-R1, -C(O)O⁻, -C(O)-OR1, -CF₃, -SO₃R1 et sulfonate -SO₃⁻ et R1 est un groupement alkyl linéaire ou ramifié en C1-C8.

Avantageusement, le catalyseur à base de cuivre comprend en outre du (2,2,6,6-tétraméthylpipéridin-1-yl)oxyl (TEMPO) ou un dérivé tel que l'hydroxy-TEMPO, l'amino-TEMPO ou l'acétamido-TEMPO.

Selon un mode de réalisation avantageux, le procédé est caractérisé en ce que le catalyseur à base de cuivre comprend en outre une base choisie dans le groupe comprenant le 1,8-diazabicyclo[5.4.0]undéc-7-ène (DBU), le 1,5-diazabicyclo[4.3.0]non-5-ène (DBN), le 1-méthylimidazole (NMI) et un sel d'acétate, en particulier un acétate de sodium ou un acétate de potassium.

Avantageusement encore, le procédé selon l'invention est caractérisé en ce que le catalyseur à base de cuivre comprend une bipyridine en particulier la 2,2'-bipyridine.

Avantageusement le procédé est caractérisé en ce que la phase liquide organique apolaire (A) est choisie dans le groupe comprenant un alcane en C5-C8, en particulier un alcane linéaire, plus particulièrement encore l'hexane.

Selon un mode avantageux de réalisation, le procédé selon l'invention est caractérisé en ce que la phase liquide polaire (B) est choisie dans le groupe comprenant l'acétonitrile, le diméthylsulfoxide (DMSO), le sulfolane, un sel de 1-(C1-C6)-alkyl-3-méthyl imidazolium et un sel de 1-(C1-C6)-Alkyl-2,3-dimethyl imidazolium, et leurs mélanges.

De manière avantageuse, le contre-ion du sel est un contre-ion fluoré, en particulier choisi parmi le trifluorométhylsulfonate (triflate), l'hexafluorophosphate et le tétrafluoroborate.

Selon un mode de réalisation particulier et préféré, le catalyseur à base de cuivre est un sel de cuivre II, avantageusement choisi dans le groupe comprenant les halogénures de cuivre II et les carboxylates de cuivre II.

De manière avantageuse, l'halogénure de cuivre est choisi parmi CuI₂, CuCl₂ et CuBr₂ ; le carboxylate de cuivre est choisi parmi l'acétate de cuivre Cu(OAc)₂ et l'acétyl acétonate de cuivre II Cu(Acac)₂

Selon un mode de réalisation particulièrement avantageux, le procédé selon l'invention est caractérisé en ce que l'étape a) est réalisée dans un réacteur agité de type réacteur à échange de chaleur.

Dans un mode de réalisation particulier, la réaction d'oxydation continue est réalisé via co-alimentation du réacteur continu avec les deux solutions, l'une contenant le réactif et l'autre contenant le catalyseur.

Ainsi, dans un mode de réalisation particulier, le procédé selon l'invention est caractérisé en ce qu'il comprend les étapes concomitantes suivantes :
a. co-alimentation d'un réacteur continu sous pression d'oxygène comprise entre 1 et 30 bar avec l'alcool de formule (I) en solution dans une phase liquide organique apolaire (A) de densité strictement inférieure à 0,7 et avec le catalyseur à base de cuivre en solution dans une phase liquide polaire (B) de densité supérieure ou égale à 0,75, afin de réaliser une oxydation de l'alcool (I) ;
b. décompression et séparation liquide/liquide de la phase liquide polaire (B) contenant le catalyseur et de la phase liquide organique apolaire (A) contenant le produit (II) ;
c. récupération du produit (II) en solution dans phase liquide organique apolaire (A) supérieure ;
d. optionnellement, évaporation de la phase liquide organique apolaire (A) pour récupérer le produit (II).

Selon le mode de réalisation comprenant la co-alimentation du réacteur continu avec les deux solutions, le procédé est aussi caractérisé en ce que tout ou partie de la phase liquide polaire (B) contenant le catalyseur séparée au niveau de l'étape b) est réintroduite au niveau de l'étape de co-alimentation a).

De manière avantageuse encore, le mode de réalisation comprenant la co-alimentation du réacteur avec les deux solutions est caractérisé en ce que le ratio molaire entre le composé (I) et le catalyseur à base de cuivre est compris entre 10 :1 et 20 :1 au niveau de l'étape de co-alimentation a).

Dans un mode de réalisation alternatif du procédé selon l'invention, on prévoit la préparation d'un mélange biphasique homogène comprenant l'alcool de formule (I) en solution dans une phase liquide organique apolaire (A) de densité strictement inférieure à 0,7 et le catalyseur à base de cuivre en solution dans une phase liquide polaire (B) de densité supérieure ou égale à 0,75.

Les deux phases n'étant pas miscibles, c'est ainsi ce mélange biphasique qui va alimenter un réacteur continu sous pression d'oxygène au sein duquel l'oxydation en continu de l'alcool (I) est réalisée.

Selon ce mode de réalisation particulier prévoyant la préparation d'un mélange préalable biphasique homogène, on prévoit une boucle de recirculation entre le mélangeur M dans lequel est maintenu agité le mélange biphasique homogène et le réacteur continu sous pression dans lequel est réalisé la réaction d'oxydation. Cette boucle de recirculation permet d'épuiser le milieu en alcool (I) et de l'enrichir en l'aldéhyde formé (II) ; cette boucle de recirculation opère jusqu'à ce que le mélange biphasique soit substantiellement épuisé en l'alcool (I), c'est-à-dire qu'il soit substantiellement totalement oxydé en l'aldéhyde (II).

Par l'expression « mélange biphasique homogène », on entend ici que l'agitation dans le mélangeur permet de créer et maintenir un mélange dans lequel les deux phases (A et B) bien que non miscibles sont uniformément réparties l'une dans l'autre et ne sont pas distinguables à l'œil nu et ne se séparent pas au sein du mélangeur. Un mélange biphasique homogène se caractérise par le fait que si l'on prélève un échantillon dudit mélange, il contient une quantité substantiellement égale des deux phases.

Ainsi dans le cas d'un mode de réalisation du procédé selon l'invention avec la préparation d'un mélange biphasique homogène, ledit procédé est caractérisé en ce qu'il comprend les étapes concomitantes suivantes :
a. préparation, dans un mélangeur M, d'un mélange biphasique homogène comprenant l'alcool de formule (I) en solution dans une phase liquide organique apolaire (A) de densité strictement inférieure à 0,7 et le catalyseur à base de cuivre en solution dans une phase liquide polaire (B) de densité supérieure ou égale à 0,75 ;
b. alimentation d'un réacteur continu sous pression d'oxygène comprise entre 1 et 30 bar avec le mélange biphasique homogène pour réaliser l'oxydation de l'alcool (I) ;
c. réalisation d'une boucle de recirculation entre le réacteur agité et le mélangeur M jusqu'à conversion substantiellement totale de l'alcool (I) en aldéhyde (II) ;
d. décompression et séparation liquide/liquide de la phase liquide polaire (B) contenant le catalyseur et de la phase liquide organique apolaire (A) contenant le produit (II) ;
e. récupération du produit (II) en solution dans phase liquide organique apolaire (A) ;
f. optionnellement, évaporation de la phase liquide organique apolaire (A) pour récupérer le produit (II).

Selon un mode de réalisation particulier du procédé selon le mode de réalisation ci-avant, avec réalisation d'une préparation d'un mélange biphasique homogène, l'oxygène qui n'a pas réagi dans le réacteur continu, en particulier un réacteur continu H.E.R (Heat Exchange Reactor - réacteur à échange de chaleur) est décompressé en sortie dudit réacteur, capté, recompressé et réinjecté dans ledit réacteur au niveau de d'alimentation.

Selon un mode de réalisation particulier du procédé selon l'un des modes de réalisation tel que décrit ci-avant, le réacteur continu est un réacteur HER (Heat Exchange Reactor ou Réacteur à Echange de Chaleur).

Le procédé selon l'invention est caractérisé par le fait qu'il est réalisé en continu dans un solvant polaire en présence d'un catalyseur à base de cuivre peu onéreux sous une pression d'air supérieure à 1 bar et inférieure à 30 bar, et à une température comprise entre 30 et 200°C, en particulier entre 40 et 180°C.

Le temps de séjour dans le réacteur d'oxydation est avantageusement inférieur à 240 minutes, plus avantageusement le temps de séjour est compris entre 5 min et 80 minutes.

Ce procédé offre l'avantage d'une grande sélectivité de la réaction à des coûts réduits.

Le procédé complet d'oxydation en solution en continu comprend de manière générale trois grandes étapes ou zones :
- Etape/Zone 1 : étape de préparation du catalyseur et de l'alcool ;
- Etape/Zone 2 : étape d'oxydation de l'alcool (I) ;
- Etape/Zone 3 : étape de récupération de l'aldehyde (II).

### Etape/Zone 1 : Préparation du catalyseur

Le catalyseur utilisé est obtenu en mélangeant un équivalent d'un sel de cuivre(II) tels que les halogénures de cuivre, en particulier CuI₂, le triflate de cuivre, l'acétate de cuivre, l'acétyle acétonate de cuivre, l'hydroxyde de cuivre avec un solvant ou un mélange de solvants polaire dense, afin d'obtenir une phase liquide polaire (B) de densité supérieure ou égale à 0,75, en particulier supérieur ou égale à 0,8, voire supérieure ou égale à 0,9 voire encore supérieure ou égale à 1.

Des solvants adaptés sont l'acétonitrile, le diméthylsulfoxyde (DMSO), le sulfolane, un sel de 1-(C1-C6)-alkyl-3-méthyl imidazolium et un sel de 1-(C1-C6)-alkyl-2,3-diméthyl imidazolium, et leurs mélanges. Et de manière avantageuse, le contre-ion du sel est un contre-ion fluoré, en particulier choisi parmi le trifluorométhylsulfonate (triflate), l'hexafluorophosphate et le tétrafluoroborate l'acétonitrile ou de préférence des liquides ioniques tels que les sels de 1-alkyl,3 méthyl imidazolium ou de 1-alkyl-2,3-diméthyl imidazolium.

Le catalyseur ainsi obtenu est à une concentration comprise entre 0,01M et 1M.

Peut être ajouté, entre 1 et 4, voire entre 1,8 et 2,5 équivalent molaire de ligand de formule générale : 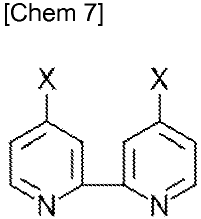 où X est choisi dans le groupe comprenant -C(O)-R1, -C(O)O⁻, -C(O)-OR1, -CF3, -SO3R1 et sulfonate -SO₃⁻ et R1 est un groupement alkyl linéaire ou ramifié en C1-C8.

De plus de 0,5 à 2 équivalent, en particulier 1 équivalent, de (2,2,6,6-tétraméthylpipéridin-1-yl)oxyl (TEMPO), ou un dérivé de celui-ci peut être ajouté.

Enfin, peuvent encore être ajouté, entre 0 et 4 équivalent, en particulier entre 1 et 3, voire entre 1 et 2,2 équivalents d'une base choisie dans le groupe comprenant le 1,8-diazabicyclo[5.4.0]undéc-7-ène (DBU), le 1,5-diazabicyclo[4.3.0]non-5-ène (DBN), le 1-méthylimidazole (NMI) et un sel d'acétate, en particulier un acétate de sodium ou un acétate de potassium.

Le mélange contenant l'alcool (I) est réalisé de manière classique en mélangeant l'alcool (i) dans un solvant organique apolaire de manière à obtenir une phase liquide organique apolaire (A) de densité strictement inférieure à 0,7, voire inférieure à 0,6. La concentration de l'alcool dans ladite phase liquide organique apolaire est comprise entre 0,1M et 10M, de préférence entre 0,1 et 1M.

### Etape/Zone 2 : étape d'oxydation de l'alcool (I)

Comme évoqué précédemment, l'étape d'oxydation continu peut être réalisée de deux manières/façons différentes. Soit par co-alimentation d'un réacteur continu avec les deux phases, soir par l'alimentation dudit réacteur continu avec un mélange biphasique homogène préalablement préparé et maintenu sous agitation dans un mélangeur. La mise en œuvre du procédé peut se faire de 2 façons :

### Façon n°1 :

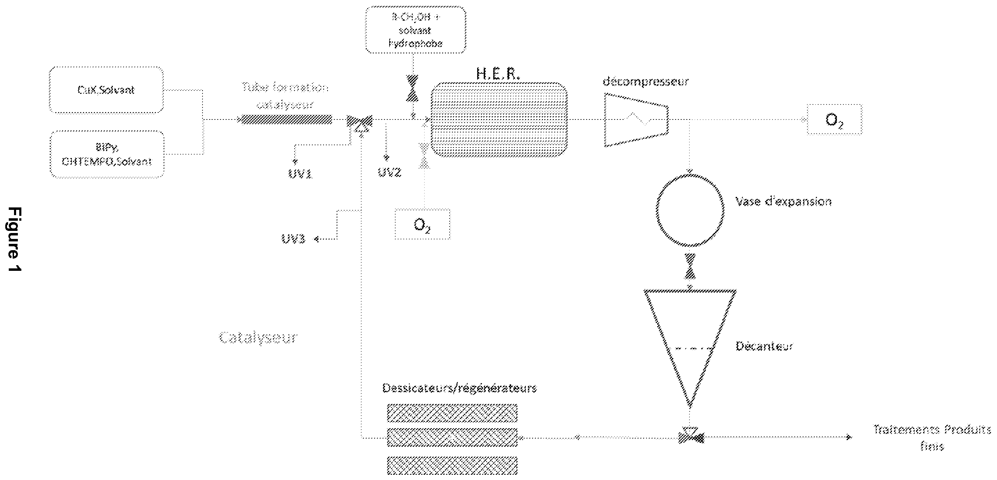

Le mélange catalyseur est préparé dans un réacteur agité ou dans un tube réactionnel comme évoqué plus avant puis pompé vers la zone de réaction continue dans un réacteur continu, en particulier de type H.E.R. (pour Heat Exchange Reactor). A l'entrée du réacteur continu, sont aussi injectées la phase contenant l'alcool (I) et l'oxygène sous pression comprise entre 1 et 30 bars. Un tel mode de réalisation est illustré sur la Figure 1.

Les injections sont pilotées par des débimètres à des flux notés F_{cataneuf}, F_{substrat} et F_{O2} de manière à ce que la conversion en sortie du H.E.R. soit supérieure à 99%.

La réaction a lieu dans le réacteur continu à une température comprise entre 20°C et 200°C et le produit de la réaction arrive dans la zone 3 de séparation du catalyseur, des gaz et de la solution contenant l'aldéhyde (II).

Le réacteur continu est alimenté en continu en la phase contenant l'alcool (I), en la phase contenant le système catalytique et oxygène.

Le flux de catalyseur réinjecté (F_{catarecyc}) se fait dans le flux de catalyseur frais et les deux flux sont pilotés de manière à ce que la molarité entre le substrat alcool (I) et le catalyseur (combinant le flux de catalyseur frais et le flux de catalyseur recyclé) soit maintenu un ratio compris entre 10/1 et 20/1. Lorsque les flux F_{substrat} et F_{O2} sont constants alors F_{cataneuf}+F_{catarecyc} est constant. Dès lors, le taux de catalyseur est piloté par 3 détecteurs UV : UV1 permet de connaître la concentration de catalyseur frais, UV3 permet de connaître la concentration de catalyseur recyclé, UV2 permet de contrôler que la concentration de catalyseur voulue est bien injectée en continu dans le réacteur.

On rappellera que dans un réacteur continu, la concentration dans le milieu réactionnel correspond à la concentration en sortie de réacteur.

La pression de fonctionnement du réacteur est comprise avantageusement entre 1 bar et 200 bars, plus avantageusement entre 1 et 100 bars. Dans certains modes de réalisation, la pression de fonctionnement du réacteur est comprise entre 1 bar et 50 bars.

La température d'oxydation est avantageusement entre 15 et 100°C, particulièrement entre 20 et 80°C. La température d'oxydation est bien entendu inférieure à la température de décomposition du produit.

La température d'oxydation est avantageusement maintenue constante. Tout moyen connu de l'homme du métier peut être utilisé à cette fin. À titre d'exemple, nous pouvons citer l'échangeur de chaleur à l'intérieur du réacteur, à l'extérieur, par maîtrise de la température d'alimentation.

Le réacteur continu peut avantageusement être muni de moyens d'agitation tels que des mélangeurs statiques. En effet, une agitation suffisante permet de garantir un bon niveau de mélangeage et ainsi éviter les zones mortes ou ségrégation du milieu réactionnel.

De manière préférée le réacteur continu est un réacteur continu de type HER (Heat Exchange Reactor) tel que commercialisé par la société Khimod.

Par définition, un réacteur continu a au moins une entrée et au moins une sortie qui sont systématiquement ouvertes. Comme connu de l'homme du métier, la sortie du réacteur doit être suffisamment éloignée de l'entrée pour éviter les problèmes de chemin préférentiel. Idéalement, l'entrée et la sortie du réacteur sont éloignées au maximum.

Dans le cas d'un réacteur bi-phasique, la sortie est bien entendu placée en contact avec la phase liquide.

L'effluent du réacteur agité est envoyé à l'étape de récupération du produit d'oxydation.

### Etape/Zone 3 : étape de récupération de l'aldéhyde (II)

Cette zone 3 se caractérise d'abord par un décompresseur qui évacue l'oxygène permettant son recyclage éventuel, puis par un décanteur continu qui pompe la solution plus dense contenant le catalyseur par le bas du décanteur et envoie par débordement vers la zone/étape 1 en passant par un dessiccateur (thermique ou chimique).

La récupération de l'aldéhyde de la solution et la séparation de son solvant peuvent se faire selon toute méthode connue de l'homme de l'art, de telle sorte à l'isoler et l'amener à un taux de matières volatiles inférieur à 1% en poids.

### Façon n°2 :

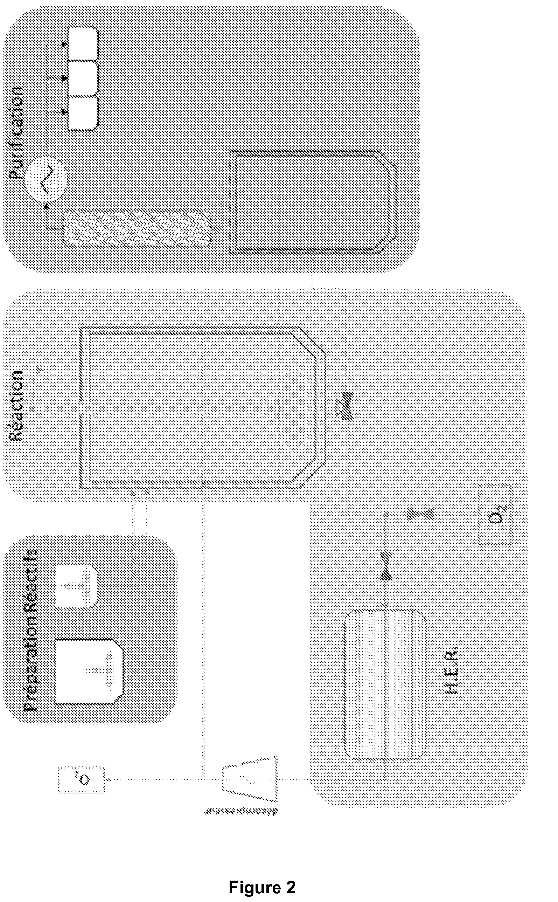

Une deuxième façon de mettre en œuvre le procédé comprend la réalisation d'un mélange biphasique homogène comprenant d'une part le catalyseur dans un phase, et d'autre part le réactif alcool (I) dans une autre phase, les deux phases n'étant pas miscibles. Un tel mode de réalisation est illustré sur la Figure 2.

Dans ce cas un mélange de solutions du catalyseur et du réactif R-CH₂OH est préparé dans un mélangeur, lui-même connecté au réacteur continu, par exemple un réacteur continu de type H.E.R., par une boucle de circulation à un débit D1. En amont du réacteur continu, par exemple un réacteur continu de type HER, l'oxygène est introduit à un débit D2 et à une pression comprise entre 0,2 et 30 bars. L'oxygène n'ayant pas réagi est décompressé en sortie du réacteur continu pour être éventuellement recyclé en entrée du réacteur continu, par exemple réacteur de type H.E.R. Le réacteur continu est maintenu sous agitation et la boucle de recirculation fonctionne jusqu'à la conversion totale de l'alcool (I).

Dans ce mode de réalisation, les températures du mélangeur et du réacteur continu, en particulier un réacteur H.E.R., sont de préférence les mêmes comprises et de préférence entre 15 et 80°C, en particulier entre 20 et 60°C.

L'intérêt du procédé selon la façon 1 ou la façon 2 est d'une part, de permettre une conversion totale d'un alcool en aldéhyde, en particulier une phéromone porteuse de la fonction aldéhyde avec un degré de pureté très satisfaisant.

D'autre part, ces deux façons de procéder permettent d'éviter d'avoir recours à des réacteurs de volume important supportant de forte pression, la phase sous pression de gaz se bornant à la partie de la réaction ayant lieu dans le réacteur continu sous pression, en particulier un réacteur de type HER.

### Légende des Figures.

Figure 1 : réalisation du procédé avec co-alimentation du réacteur avec les deux phases.
Figure 2 : réalisation du procédé avec préparation préalable d'un mélange biphasique homogène dans un mélangeur alimentant en continu un réacteur, avec boucle de recirculation entre le réacteur et le mélangeur.

### Exemples :

Les matières premières (CuI₂, bipyridine, TEMPO) et solvants sont trouvées commercialement chez Sigma Aldrich.

Le Z11-hexadécénol est produit selon une méthode connue de l'homme du métier sur le site de Salin de Giraud (M2I Development) et présente une pureté de 92% en poids. L'impureté principale (3,2%) est le E11-hexadécénal.

Le réacteur H.E.R. est fabriqué et fourni par la société Khimod.

### Exemple 1 : Mode de réalisation avec préparation d'un mélange biphasique homogène.

Dans un réacteur de 10L maintenu sous vive agitation, on prépare 480 g de Z11-hexadécénol dans 3,3 L d'hexane puis on ajoute 3L d'une solution d'acétonitrile contenant :
- 19 g de iodure de cuivre (CuI₂),
- 15,6g de bipyridine,
- 16,4 g de N-méthyl imidazole,
- 15,6 g de TEMPO.

Le mélange biphasique est agité de manière à avoir une distribution homogène des deux phases.

La solution est pompée vers le HER au moyen d'un pompe haute pression à raison de 50 mL/mn en même temps que de l'oxygène qui est introduit sous 12 bars à raison de 1L/mn. L'ensemble du système est maintenu à 25°C.

Des prélèvements réguliers sont pratiqués dans le réacteur de 1L et la réaction est arrêtée quand la conversion du Z11-hexadécénol est complète au bout de 10 heures.

A la fin de la réaction la recirculation est stoppée ainsi que l'agitation. La phase inférieure est évacuée pour être éventuellement recyclée (cf. exemple 3). La phase supérieure est gardée dans le réacteur, lavée 2 fois à l'eau distillée puis le solvant est évaporé sous vide pour récupérer 456 g de Z11-hexadécenal (pureté 92,0%). Il est intéressant de remarquer que dans le produit initial 3,4% de E11-hexadécenol étaient présents et que l'on retrouve 3,5% de E11-hexadécenal dans le produit final.

### Exemple 2 : Mode de réalisation avec co-alimentation du réacteur par les deux phases, sans recyclage du catalyseur

Dans un réacteur de 5L maintenu sous vive agitation, on prépare 0,564L de Z11-hexadécénol dans 1,436L d'hexane (concentration de 1 Mol/L).

Dans un autre réacteur de 5L on prépare 2L d'une solution d'acétonitrile contenant :
- 19 g de iodure de cuivre (CuI₂),
- 15,6g de bipyridine,
- 16,4 g de N-méthyl imidazole,
- 15,6 g de TEMPO.

Les deux solutions sont pompées au moyen de pompes HPLC dans le réacteur H.E.R. à des débits de 4,2ml/mn pour chaque solution. Le ratio molaire entre le catalyseur au cuivre et l'alcool est maintenant de 0,02. L'oxygène est introduit sous 12 bars à un débit de 0,2L/min. Et le produit de réaction est récupéré après décompression dans un décanteur de type ampoule à décanter de 10L. Le temps de séjour est de 2 heures pour une durée totale de réaction de 4 heures. A la fin les deux phases sont séparées (phase bleutée contenant le catalyseur en bas), puis la phase organique est lavée jusqu'à une décoloration totale. L'hexane est évaporé et on obtient 460g d'hexadécénal pur à 93% en poids.

Les résultats sont analogues à ceux de l'exemple 1.

### Exemple 3 : Mode de réalisation avec co-alimentation du réacteur par les deux phases et avec recyclage du catalyseur.

Dans un réacteur de 5L maintenu sous vive agitation, on prépare 0,564L de Z11-hexadécénol dans 1,436L d'hexane (concentration de 1 Mol/L).

Dans un autre réacteur de 2L on prépare 1 L d'une solution de 1-butyl-2,3-diméthyl imidazolium d'hexafluorophosphate contenant :
- 38 g de iodure de cuivre (CuI₂),
- 31,2g de bipyridine,
- 32,8 g de N-méthyl imidazole,
- 31,2 g de TEMPO.

Les deux solutions sont pompées au moyen de pompes HPLC dans le réacteur H.E.R. Le débit de la solution de réactif est 42mL/mn pour les 2 réactifs. Le ratio molaire entre le catalyseur au cuivre et l'alcool est maintenant de 0,22.

Le produit de réaction est récupéré après décompression dans un décanteur de type ampoule à décanter de 10L. La phase inférieure est elle-même pompée en continu pour réalimenter la réserve de catalyseur.

L'oxygène est introduit sous 12 bars à un débit de 2L/mn.

Le temps de séjour est de 24 minutes pour une durée totale de réaction de 48 min. On récupère après lavage et évaporation de l'hexane 447 g de Z11-hexadecenal d'une pureté de 91,8% en poids.

### Exemple 4 : Mode de réalisation avec co-alimentation du réacteur par les deux phases et avec recyclage du catalyseur.

Dans un réacteur de 50L maintenu sous vive agitation, on prépare 5,6L de Z11-hexadécénol dans 14L d'hexane (concentration de 1 Mol/L).

Dans un autre réacteur de 2L on prépare 1 L d'une solution de 1-butyl-2,3-diméthyl imidazolium d'hexafluorophosphate contenant :
- 38 g de iodure de cuivre (CuI₂),
- 31,2g de bipyridine,
- 32,8 g de N-méthyl imidazole,
- 31,2 g de TEMPO.

Les deux solutions sont pompées au moyen de pompes HPLC dans le réacteur H.E.R. Le débit de la solution de réactif est 42mL/mn pour les 2 réactifs. Le ratio molaire entre le catalyseur au cuivre et l'alcool est de 0,22. Et le produit de réaction est récupéré après décompression dans un décanteur de type ampoule à décanter de 10L. La phase inférieure est elle-même pompée en continu pour réalimenter la réserve de catalyseur. La phase organique est régulièrement pompée par le haut de l'ampoule dans un bac tampon de 50L.

L'oxygène est introduit sous 12 bars à un débit de 2L/mn.

Le temps de séjour est de 24 minutes pour une durée totale de réaction totale de 8 heures.

Après lavage et évaporation des phases organiques on obtient 4,56 kg de Z11-hexadecenal à 92.3% poids.

## Revendications

1. Procédé de préparation d'un aldéhyde de formule générale (II) : 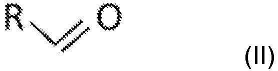
où R est une chaine hydrocarbonée linéaire de formule CₙH₂ₙ₋₂ₚ₊₁, avec :
- n qui désigne un entier naturel allant de 9 à 24,
- p correspondant au nombre d'insaturations de la chaîne hydrocarbonée qui est un nombre entier allant de 1 à 4 ;
ledit procédé est continu et comprend les étapes concomitantes suivantes :
a) alimenter un réacteur continu sous pression d'oxygène comprise entre 1 et 30 bar avec :
i) un alcool de formule générale (I) : 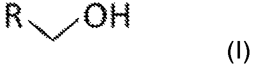 où R, n et p sont tels que définis précédemment pour le composé de formule (II), en solution dans une phase liquide organique apolaire (A) de densité strictement inférieure à 0,7 ,
ii) un catalyseur à base de cuivre en solution dans une phase liquide polaire (B) de densité supérieure ou égale à 0.75,
les phases (A) et (B) étant non miscibles l'une avec l'autre,
le ratio molaire alcool/catalyseur à base de cuivre variant de 0,01 à 0,5,
b) récupérer l'aldéhyde dans la phase (A) par séparation liquide/liquide.

2. Procédé selon la revendication 1, **caractérisé en ce que** le catalyseur à base de cuivre comprend en outre au moins un ligand du cuivre de formule générale : 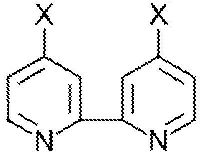 où X est choisi dans le groupe comprenant -C(O)-R1, -C(O)O⁻, -C(O)-OR1, -CF₃, -SO₃R1 et sulfonate -SO₃⁻ et R1 est un groupement alkyl linéaire ou ramifié en C1-C8.

3. Procédé selon la revendication 1 ou 2, **caractérisé en ce que** le catalyseur à base de cuivre comprend en outre du (2,2,6,6-tétraméthylpipéridin-1-yl)oxyl (TEMPO) ou un dérivé tel que l'hydroxy-TEMPO, l'amino-TEMPO ou l'acétamido-TEMPO.

4. Procédé selon l'une des revendications 1 à 3, **caractérisé en ce que** le catalyseur à base de cuivre comprend en outre une base choisie dans le groupe comprenant le 1,8-diazabicyclo[5.4.0]undéc-7-ène (DBU), le 1,5-diazabicyclo[4.3.0]non-5-ène (DBN), le 1-méthylimidazole (NMI) et un sel d'acétate, en particulier un acétate de sodium ou un acétate de potassium.

5. Procédé selon l'une des revendications précédentes, **caractérisé en ce que** le catalyseur à base de cuivre comprend une bipyridine, en particulier la 2,2'-bipyridine.

6. Procédé selon l'une des revendications précédentes, **caractérisé en ce que** la phase liquide organique apolaire (A) est choisie dans le groupe comprenant un alcane en C5-C8, en particulier un alcane linéaire, en particulier l'hexane.

7. Procédé selon l'une des revendications précédentes, **caractérisé en ce que** la phase liquide polaire (B) est choisie dans le groupe comprenant l'acétonitrile, le diméthylsulfoxide (DMSO), le sulfolane, un sel de 1-(C1-C6)-alkyl-3-méthyl imidazolium et un sel de 1-(C1-C6)-alkyl-2,3-diméthyl imidazolium, et leurs mélanges.

8. Procédé selon la revendication 7, **caractérisé en ce que** le contre-ion du sel est un contre-ion fluoré, en particulier choisi parmi le trifluorométhylsulfonate (triflate), l'hexafluorophosphate et le tétrafluoroborate.

9. Procédé selon l'une des revendications précédentes, **caractérisé en ce que** le catalyseur à base de cuivre est un sel de cuivre II, avantageusement choisi dans le groupe comprenant les halogénures de cuivre II et les carboxylates de cuivre II.

10. Procédé selon la revendication 9, **caractérisé en ce que** l'halogénure de cuivre est choisi parmi CuI₂, CuCl₂ et CuBr₂ ; le carboxylate de cuivre est choisi parmi l'acétate de cuivre Cu(OAc)₂ et l'acétyl acétonate de cuivre II Cu(Acac)₂.

11. Procédé selon l'une des revendications précédentes, **caractérisé en ce que** l'étape a) est réalisée dans un réacteur continu de type réacteur à échange de chaleur.

12. Procédé selon l'une des revendications précédentes, **caractérisé en ce qu'**il comprend les étapes concomitantes suivantes :
a. co-alimentation d'un réacteur continu sous pression d'oxygène comprise entre 1 et 30 bar avec l'alcool de formule (I) en solution dans une phase liquide organique apolaire (A) de densité strictement inférieure à 0,7 et avec le catalyseur à base de cuivre en solution dans une phase liquide polaire (B) de densité supérieure ou égale à 0,75, afin de réaliser une oxydation de l'alcool (I) ;
b. décompression et séparation liquide/liquide de la phase liquide polaire (B) contenant le catalyseur et de la phase liquide organique apolaire (A) contenant le produit (II) ;
c. récupération du produit (II) en solution dans la phase liquide organique apolaire (A) supérieure ;
d. optionnellement, évaporation de la phase liquide organique apolaire (A) pour récupérer le produit (II).

13. Procédé selon la revendication 12, **caractérisé en ce que** tout ou partie de la phase liquide polaire (B) contenant le catalyseur séparée est réintroduite au niveau de l'étape de co-alimentation a).

14. Procédé selon l'une des revendications 12 et 13, **caractérisé en ce que** le ratio molaire entre le composé (I) et le catalyseur à base de cuivre est compris entre 10 :1 et 20 :1 au niveau de l'étape de co-alimentation.

15. Procédé selon l'une des revendications 1 à 10, **caractérisé en ce qu'**il comprend les étapes concomitantes suivantes :
a. préparation, dans un mélangeur M, d'un mélange biphasique homogène comprenant l'alcool de formule (I) en solution dans une phase liquide organique apolaire (A) de densité strictement inférieure à 0,7 et le catalyseur à base de cuivre en solution dans une phase liquide polaire (B) de densité supérieure ou égale à 0,75 ;
b. alimentation d'un réacteur continu sous pression d'oxygène comprise entre 1 et 30 bar avec le mélange biphasique homogène afin de réaliser l'oxydation de l'alcool (I) ;
c. réalisation d'une boucle de recirculation entre le réacteur agité et le mélangeur M jusqu'à conversion substantiellement totale de l'alcool (I) en aldéhyde (II) ;
d. décompression et séparation liquide/liquide de la phase liquide polaire (B) contenant le catalyseur et de la phase liquide organique apolaire (A) contenant le produit (II) ;
e. récupération du produit (II) en solution dans phase liquide organique apolaire (A) ;
f. optionnellement, évaporation de la phase liquide organique apolaire (A) pour récupérer le produit (II).

16. Procédé selon la revendication 15, **caractérisé en ce que** l'oxygène qui n'a pas réagi dans le réacteur est décompressé en sortie dudit réacteur, capté, recompressé et réinjecté dans ledit réacteur au niveau de d'alimentation.

## Patentansprüche

1. Verfahren zur Herstellung eines Aldehyds der allgemeinen Formel (II): 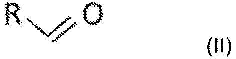
wobei R eine lineare Kohlenwasserstoffkette der Formel CₙH₂ₙ₋₂ₚ₊₁ ist, mit:
- n als natürliche Ganzzahl von 9 bis 24,
- p als Anzahl der Ungesättigtheiten der Kohlenwasserstoffkette, die eine Ganzzahl von 1 bis 4 ist;
wobei das Verfahren kontinuierlich ist und die folgenden begleitenden Schritte umfasst:
a) Beschicken eines kontinuierlichen Reaktors unter Sauerstoffdruck zwischen 1 und 30 bar mit:
i) einem Alkohol der allgemeinen Formel (I): 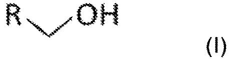 wobei R, n und p wie zuvor für die Verbindung der Formel (II) definiert sind, gelöst in einer apolaren organischen flüssigen Phase (A) mit einer Dichte von strikt unter 0,7,
ii) einem Katalysator auf Kupferbasis gelöst in einer polaren flüssigen Phase (B) mit einer Dichte von über oder gleich 0,75,
wobei die Phasen (A) und (B) nicht miteinander mischbar sind,
wobei das molare Verhältnis Alkohol/Katalysator auf Kupferbasis zwischen 0,01 und 0,5 schwankt,
b) Rückgewinnen des Aldehyds in der Phase (A) durch Flüssig-Flüssig-Trennung.

2. Verfahren nach Anspruch 1, **dadurch gekennzeichnet, dass** der Katalysator auf Kupferbasis ferner mindestens einen Kupferliganden der allgemeinen Formel umfasst: 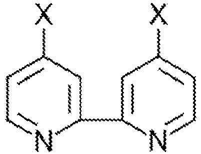 wobei X aus der Gruppe ausgewählt ist, die -C(O)-R1, -C(O)O⁻, -C(O)-OR1, -CF₃, -SO₃R1 und Sulfonat -SO₃⁻ umfasst und R1 eine lineare oder verzweigte C1-C8-Alkylgruppe ist.

3. Verfahren nach Anspruch 1 oder 2, **dadurch gekennzeichnet, dass** der Katalysator auf Kupferbasis ferner (2,2,6,6-Tetramethylpiperidin-1-yl)oxyl (TEMPO) oder ein Derivat wie Hydroxy-TEMPO, Amino-TEMPO oder Acetamido-TEMPO umfasst.

4. Verfahren nach einem der Ansprüche 1 bis 3, **dadurch gekennzeichnet, dass** der Katalysator auf Kupferbasis ferner eine Base umfasst, die aus der Gruppe ausgewählt ist, die 1,8-Diazabicyclo[5.4.0]undec-7-en (DBU), 1,5-Diazabicyclo[4.3.0]non-5-en (DBN), 1-Methylimidazol (NMI) und ein Acetatsalz, insbesondere ein Natriumacetat oder ein Kaliumacetat, umfasst.

5. Verfahren nach einem der vorhergehenden Ansprüche, **dadurch gekennzeichnet, dass** der Katalysator auf Kupferbasis ein Bipyridin, insbesondere 2,2'-Bipyridin, umfasst.

6. Verfahren nach einem der vorhergehenden Ansprüche, **dadurch gekennzeichnet, dass** die apolare organische flüssige Phase (A) aus der Gruppe ausgewählt ist, die ein C5-C8-Alkan, insbesondere ein lineares Alkan, insbesondere Hexan, umfasst.

7. Verfahren nach einem der vorhergehenden Ansprüche, **dadurch gekennzeichnet, dass** die polare flüssige Phase (B) aus der Gruppe ausgewählt ist, die Acetonitril, Dimethylsulfoxid (DMSO), Sulfolan, ein Salz von 1-(C1-C6)-Alkyl-3-methylimidazolium und ein Salz von 1-(C1-C6)-Alkyl-2,3-dimethylimidazolium und deren Mischungen umfasst.

8. Verfahren nach Anspruch 7, **dadurch gekennzeichnet, dass** das Gegenion des Salzes ein fluoriertes Gegenion ist, insbesondere ausgewählt aus Trifluormethylsulfonat (Triflat), Hexafluorophosphat und Tetrafluoroborat.

9. Verfahren nach einem der vorhergehenden Ansprüche, **dadurch gekennzeichnet, dass** der Katalysator auf Kupferbasis ein Kupfer(II)-Salz ist, das in vorteilhafter Weise aus der Gruppe ausgewählt ist, die Kupfer(II)-Halogenide und Kupfer(II)-Carboxylate umfasst.

10. Verfahren nach Anspruch 9, **dadurch gekennzeichnet, dass** das Kupferhalogenid aus Cul₂, CuCl₂ und CuBr₂ ausgewählt ist; das Kupfercarboxylat aus Kupferacetat Cu(OAc)₂ und Kupfer(II)-acetylacetonat Cu(Acac)₂ ausgewählt ist.

11. Verfahren nach einem der vorhergehenden Ansprüche, **dadurch gekennzeichnet, dass** der Schritt a) in einem kontinuierlichen Reaktor vom Typ eines Wärmeaustauschreaktors durchgeführt wird.

12. Verfahren nach einem der vorhergehenden Ansprüche, **dadurch gekennzeichnet, dass** es die folgenden begleitenden Schritte umfasst:
a. Co-Beschicken eines kontinuierlichen Reaktors unter Sauerstoffdruck zwischen 1 und 30 bar mit dem Alkohol der Formel (I) gelöst in einer apolaren organischen flüssigen Phase (A) mit einer Dichte von strikt unter 0,7 und mit dem Katalysator auf Kupferbasis gelöst in einer polaren flüssigen Phase (B) mit einer Dichte von über oder gleich 0,75, um eine Oxidation des Alkohols (I) durchzuführen;
b. Dekomprimieren und Flüssig-Flüssig-Trennung der polaren flüssigen Phase (B), die den Katalysator enthält, und der apolaren organischen flüssigen Phase (A), die das Produkt (II) enthält;
c. Rückgewinnen des Produkts (II) gelöst in der oberen apolaren organischen flüssigen Phase (A);
d. optional Verdampfen der apolaren organischen flüssigen Phase (A), um das Produkt (II) zurückzugewinnen.

13. Verfahren nach Anspruch 12, **dadurch gekennzeichnet, dass** die gesamte oder ein Teil der separierten polaren flüssigen Phase (B), die den Katalysator enthält, bei Schritt der Co-Beschickung a) zurückgeführt wird.

14. Verfahren nach einem der Ansprüche 12 und 13, **dadurch gekennzeichnet, dass** das molare Verhältnis zwischen der Verbindung (I) und dem Katalysator auf Kupferbasis zwischen 10:1 bis 20:1 bei Schritt der Co-Beschickung liegt.

15. Verfahren nach einem der Ansprüche 1 bis 10, **dadurch gekennzeichnet, dass** es die folgenden begleitenden Schritte umfasst:
a. Herstellen eines homogenen Zweiphasengemischs in einem Mischer M, das den Alkohol der Formel (I) gelöst in einer apolaren organischen flüssigen Phase (A) mit einer Dichte von strikt unter 0,7 und den Katalysator auf Kupferbasis gelöst in einer polaren flüssigen Phase (B) mit einer Dichte von über oder gleich 0,75 umfasst;
b. Beschicken eines kontinuierlichen Reaktors unter Sauerstoffdruck zwischen 1 und 30 bar mit dem homogenen Zweiphasengemisch, um die Oxidation des Alkohols (I) durchzuführen;
c. Herstellen einer Rezirkulationsschleife zwischen dem Rührreaktor und dem Mischer M bis zur im Wesentlichen vollständigen Umwandlung des Alkohols (I) in Aldehyd (II);
d. Dekomprimieren und Flüssig-Flüssig-Trennung der polaren flüssigen Phase (B), die den Katalysator enthält, und der apolaren organischen flüssigen Phase (A), die das Produkt (II) enthält;
e. Rückgewinnen des Produkts (II) gelöst in der apolaren organischen flüssigen Phase (A);
f. optional Verdampfen der apolaren organischen flüssigen Phase (A), um das Produkt (II) zurückzugewinnen.

16. Verfahren nach Anspruch 15, **dadurch gekennzeichnet, dass** der Sauerstoff, der im Reaktor nicht reagiert hat, am Ausgang des Reaktors dekomprimiert, aufgefangen, wieder komprimiert und in den Reaktor bei der Beschickung zurückgeführt wird.

## Claims

1. A method for preparing an aldehyde of general formula (II): 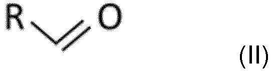
where R is a linear hydrocarbon chain of formula CₙH₂ₙ₋₂ₚ₊₁, with:
- n which is a natural number ranging from 9 to 24,
- p corresponding to the number of unsaturations of the hydrocarbon chain which is an integer ranging from 1 to 4;
said method is continuous and comprises the following concomitant steps:
a) feeding a continuous reactor under oxygen pressure of between 1 and 30 bar with:
i) an alcohol of general formula (I): 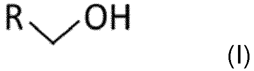 where R, n and p are as defined previously for the compound of formula (II), in solution in an apolar organic liquid phase (A) with a density strictly less than 0.7,
ii) a copper-based catalyst in solution in a polar liquid phase (B) with a density greater than or equal to 0.75,
the phases (A) and (B) being immiscible with each other,
the alcohol/copper-based catalyst molar ratio ranging from 0.01 to 0.5,
b) recovering the aldehyde in phase (A) by liquid/liquid separation.

2. The method according to claim 1, **characterized in that** the copper-based catalyst further comprises at least one copper ligand of general formula: 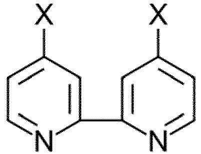 where X is selected from the group comprising -C(O)-R1, -C(O)O⁻, -C(O)-OR1, - CF₃, -SO₃R1 and sulfonate -SO₃⁻ and R1 is a linear or branched C1-C8 alkyl group.

3. The method according to claim 1 or 2, **characterized in that** the copper-based catalyst further comprises (2,2,6,6-tetramethylpiperidin-1-yl)oxyl (TEMPO) or a derivative such as hydroxy-TEMPO, amino-TEMPO or acetamido-TEMPO.

4. The method according to one of claims 1 to 3, **characterized in that** the copper-based catalyst further comprises a base selected from the group comprising 1,8-diazabicyclo[5.4.0]undec-7-ene (DBU), 1,5-diazabicyclo[4.3.0]non-5-ene (DBN), 1-methylimidazole (NMI) and an acetate salt, in particular sodium acetate or potassium acetate.

5. The method according to one of the preceding claims, **characterized in that** the copper-based catalyst comprises a bipyridine, in particular 2,2'-bipyridine.

6. The method according to one of the preceding claims, **characterized in that** the apolar organic liquid phase (A) is selected from the group comprising a C5-C8 alkane, in particular a linear alkane, in particular hexane.

7. The method according to one of the preceding claims, **characterized in that** the polar liquid phase (B) is selected from the group comprising acetonitrile, dimethylsulfoxide (DMSO), sulfolane, a salt of 1-(C1-C6)-alkyl-3-methyl imidazolium and a salt of 1-(C1-C6)-alkyl-2,3-dimethyl imidazolium, and mixtures thereof.

8. The method according to claim 7, **characterized in that** the counterion of the salt is a fluorinated counterion, in particular selected from trifluoromethylsulfonate (triflate), hexafluorophosphate and tetrafluoroborate.

9. The method according to one of the preceding claims, **characterized in that** the copper-based catalyst is a copper II salt, advantageously selected from the group comprising copper II halides and copper II carboxylates.

10. The method according to claim 9, **characterized in that** the copper halide is selected from CuI₂, CuCl₂ and CuBr₂; the copper carboxylate is selected from copper acetate Cu(OAc)₂ and copper II acetyl acetonate Cu(Acac)₂.

11. The method according to one of the preceding claims, **characterized in that** step a) is carried out in a continuous reactor of the heat exchange reactor type.

12. The method according to one of the preceding claims, **characterized in that** it comprises the following concomitant steps:
a. co-feeding a continuous reactor under oxygen pressure of between 1 and 30 bar with the alcohol of formula (I) in solution in an apolar organic liquid phase (A) of density strictly less than 0.7 and with the copper-based catalyst in solution in a polar liquid phase (B) with a density greater than or equal to 0.75, in order to carry out an oxidation of the alcohol (I);
b. decompressing and liquid/liquid separating the polar liquid phase (B) containing the catalyst and of the apolar organic liquid phase (A) containing the product (II);
c. recovering the product (II) in solution in the upper apolar organic liquid phase (A);
d. optionally, evaporating the apolar organic liquid phase (A) to recover the product (II).

13. The method according to claim 12, **characterized in that** all or part of the polar liquid phase (B) containing the separated catalyst is reintroduced at the co-feeding step a).

14. The method according to one of claims 12 and 13, **characterized in that** the molar ratio between the compound (I) and the copper-based catalyst is between 10:1 and 20:1 at the co-feeding step.

15. The method according to one of claims 1 to 10, **characterized in that** it comprises the following concomitant steps:
a. preparing, in a mixer M, a homogeneous two-phase mixture comprising the alcohol of formula (I) in solution in an apolar organic liquid phase (A) of density strictly less than 0.7 and the copper-based catalyst in solution in a polar liquid phase (B) with a density greater than or equal to 0.75;
b. feeding a continuous reactor under oxygen pressure of between 1 and 30 bar with the homogeneous two-phase mixture in order to carry out the oxidation of the alcohol (I);
c. creating a recirculation loop between the stirred reactor and the mixer M until substantially complete conversion of the alcohol (I) into aldehyde (II);
d. decompressing and liquid/liquid separating the polar liquid phase (B) containing the catalyst and of the apolar organic liquid phase (A) containing the product (II);
e. recovering the product (II) in solution in apolar organic liquid phase (A);
f. optionally, evaporating the apolar organic liquid phase (A) to recover the product (II).

16. The method according to claim 15, **characterized in that** the oxygen which has not reacted in the reactor is decompressed at the outlet of said reactor, captured, recompressed and reinjected into said reactor at the feed.
